# EUROPÄISCHE PATENTSCHRIFT

(11) **EP 4 149 402 B1**
(45) Veröffentlichungstag und Bekanntmachung des Hinweises auf die Patenterteilung: **04.02.2026**
(21) Anmeldenummer: 22714123.1
(22) Anmeldetag: 08.03.2022
(51) Int. Cl.: A61F 13/42

(54) **ANORDNUNG ZUR FLUESSIGKEITSDETEKTION**
ARRANGEMENT FOR DETECTING LIQUID
DISPOSITIF DE DÉTECTION DE LIQUIDE

(30) Priorität: 13.04.2021 DE 102021109117
(43) Veröffentlichungstag der Anmeldung: 22.03.2023
(73) Patentinhaber: RKW SE, 68309 Mannheim (DE)
(72) Erfinder: KOPLIN, Robert, 91723 Dittenheim (DE); BRANDMAIER, Florian, 83059 Kolbermoor (DE); SCHUHMANN, Michael, 90556 Cadolzburg (DE); SCHAFSTALLER, Veronica, 81477 Muenchen (DE); JÄCKEL, Sarah, 83026 Rosenheim (DE)
(74) Vertreter: Busch, Tobias
(86) Internationale Anmeldenummer: PCT/EP2022/055914
(87) Internationale Veröffentlichungsnummer: WO 2022/218608

(56) Entgegenhaltungen:
- US-A1- 2008 054 408
- US-A1- 2021 077 313

## Beschreibung

Die Erfindung betrifft eine Anordnung zur Flüssigkeitsdetektion in Einweghygieneartikel, Einweghygieneartikel für Babys und Erwachsene sind insbesondere als Windel üblich. Herkömmliche Einweghygieneartikel dieser Art besitzen einen flächigen, längserstreckten Saugkörper, der beim Anlegen des Einweghygieneartikels durch den Schritt des Trägers läuft. Der Saugkörper umfasst häufig Superabsorbermaterial.

Der Saugkörper ist auf einer Anordnung angeordnet, die eine polyolefinische Backsheetfolie umfasst. Die Anordnung soll eine textile Anmutung aufweisen. Vorzugsweise wird dazu die Backsheetfolie mit einer Vlieslage verbunden. Die Anordnung trägt den Saugkörper und dichtet ihn nach außen hin ab.

Unter dem Stichwort "intelligente" Einweghygieneartikel sind zunehmend Produkte gefragt, die in der Lage sind, deren Benutzungszustand zu erfassen.

Babys oder Demenzerkrankte können nicht den Zustand des Einweghygieneartikels selbst feststellen bzw. sich entsprechen mitteilen. Eine Kontrolle durch Hilfspersonen ist aufwendig.

Eine Lösung bieten intelligente Einweghygieneartikel, die automatisch auf die Notwendigkeit eines Wechsels hinweisen. Dies erhöht die Lebensqualität und entlastet das Pflegepersonal. Außerdem trägt eine intelligente Windel zum Umweltschutz bei, da das Potential des Einweghygieneartikels vor einer Entsorgung ausgeschöpft wird.

Vor diesem Hintergrund sind aus dem Stand der Technik verschiedene Ansätze intelligenter Einweghygieneartikel bekannt.

Die US 6 200 250 B1 betrifft eine intelligente Windel, bei der kapazitive Elektroden auf einer Trägerfolie und einer flüssigkeitsabsorbierenden Anordnung platziert sind. Die Elektroden können dabei rechteckig ausgestaltet und in Reihe geschaltet oder als Fäden ausgebildet sein. Dabei können die Elektroden in die Windel eingeklebt oder vernäht befestigt werden.

Gemäß der US 8 978 452 B2 sowie der WO 2013/022742 A1 werden Flüssigkeitssensoren mit einem passiven Resonanzschwingkreis für einen Folienabschnitt vorgeschlagen, bei dem die aufgebrachten Strukturen zum Teil von einer Flüssigkeit aufgelöst werden können.

In der US 8 978 452 B2 kann der von einem Lesegerät detektierte Zustand eines Hygieneartikels nachträglich auch drahtlos über bekannte Datenkommunikationsverbindungen wie WLAN und Bluetooth an weitere Geräte übermittelt werden.

Die DE 10 2017 125 323 A1 beschreibt einen Folienabschnitt mit zwei Leiterbahnen, die einen passiven Resonanzschwingkreis bilden. An die Leiterbahnen ist ein Lese- und Sendegerät angeschlossen. Eine Leiterbahn ist dabei als Kondensatorplatte und die andere Leiterbahn als Spule ausgebildet. Dabei sind die Leiterbahnen auf einer Trägerfolie aufgebracht, die wiederum auf einem Folienetikett angeordnet ist. Das Folienetikett ist von einer PE-Folie umschlossen und auf der Rückwand eines Hygieneartikels aufgeklebt. Dabei muss das Folienetikett in einem zusätzlichen Arbeitsschritt auf der Rückwand des Hygieneartikels angebracht werden, wobei das Volumen des Hygieneartikels außerordentlich zunimmt. Das zusätzliche Volumen kann das Tragegefühl des Hygieneartikels ungünstig beeinflussen. Darüber hinaus ist das Folienetikett selbst unwahrscheinlich komplex in seinem Aufbau und auch in der Herstellung zu verwirklichen.

In der EP 2 654 646 B1 wird ein saugfähiger Artikel beschrieben, der eine leitfähige und eine offene Leiterschleife umfasst. Eine von einem saugfähigen Kern absorbierte Flüssigkeit ändert die Impedanz der Leiterschleifen messbar. Dies funktioniert, indem die Leiterschleifen so ausgerichtet sind, dass der Strom an einem äußeren Schenkel der zweiten Leiterschleife einen Kurzschluss erzeugt.

Die EP 3 451 988 B1 und die EP 3 760 104 A1 beschreiben eine flüssigkeitsundurchlässige Unterlage mit einer nichtleitenden Isolierschicht, auf der mindestens ein Kanal für eine Sensorbahn angeordnet ist. Vorzugsweise sind in einer farbigen Verstärkungsschicht in Form eines Aufnähers Sensorbahnen angeordnet, die mit einem Datenverarbeitungsmodul einen linken und einen rechten Stromkreis zur Erkennung von Feuchtigkeitsereignissen bilden.

Gemäß EP 3 143 974 B1 kann eine Sender-Empfänger-Anordnung zur Überwachung der Absorptionsschicht lösbar an Einweghygieneartikel gekoppelt werden. Diese Anordnung kann dann mehrfach bis vielfach an Einweghygieneartikel genutzt werden. Dadurch kann der Fokus zukünftiger Entwicklungen auf die Bereitstellung eines günstigen Einweghygieneartikels gelegt werden.

Elektroden und Leiterbahnen sind aufwendig und kostenintensiv. Einweghygieneartikel unterliegen einem Preisdruck und hohen ökologischen Anforderungen, da diese nach ihrer einmaligen Benutzung unmittelbar entsorgt werden. Darüber hinaus wird auch eine möglichst einfache Handhabung angestrebt.

Die beschriebenen Erfindungen bestehen meist aus mehreren Schichten mit unterschiedlichen Eigenschaften, die miteinander verbunden werden müssen. Dadurch entstehen dicke und schwere Einweghygieneartikel. Dies fördert die Bildung von Druckstellen und Hautirritationen bei den Windeltragenden, wodurch ein unkomfortables Tragegefühl entsteht. Zudem sollten intelligente Einweghygieneartikel bezüglich ihrer haptischen Eigenschaften gegenüber herkömmlichen in nichts nachstehen.

Aufgabe der Erfindung ist es, eine einfache und kompakte Anordnung zur Flüssigkeitsdetektion zur Verfügung zu stellen. Dabei soll die Anordnung flüssigkeitsdicht sein, die geforderten mechanischen Eigenschaften erfüllen und einen angenehmen Tragekomfort gewährleisten. Weiterhin soll die Anordnung haptisch ansprechend sein. Ziel ist es, eine möglichst raschelarme Anordnung bereitzustellen, die über Weichheit und Geschmeidigkeit verfügt. Zudem soll die Anordnung leitfähige Elemente bereitstellen, die zur zuverlässigen Flüssigkeitsdetektion nötig sind und dennoch preiswert hergestellt werden können. Die Anordnung soll die Qualität des Einweghygieneartikels steigern und den Anforderungen bei deren Herstellung in modernen Prozessen genügen.

Diese Aufgabe wird erfindungsgemäß durch eine Anordnung zur Flüssigkeitsdetektion, gemäß des nebengeordneten Hauptanspruch gelöst. Bevorzugte Varianten sind den Unteransprüchen, der Beschreibung und den Beispielen zu entnehmen.

Gemäß der Erfindung umfasst die Anordnung eine Backsheetfolie mit einem leitfähigen Aufdruck, wobei das Flächengewicht des leitfähigen Aufdrucks weniger als 3 g/m² beträgt, wobei die Backsheetfolie nicht atmungsaktiv ausgebildet ist und eine Wasserdampfdurchlässigkeit von weniger als 500 g/m² in 24 h nach ASTM D6701-01 aufweist, wobei das Flächengewicht der nicht atmungsaktiven Backsheetfolie weniger als 10 g/m² beträgt oder die Backsheetfolie atmungsaktiv ausgebildet ist und eine Wasserdampfdurchlässigkeit von mehr als 500 g/m² in 24 h nach ASTM D6701-01 aufweist, wobei das Flächengewicht der atmungsaktiven Backsheetfolie weniger als 20 g/m² beträgt.

Erfindungsgemäß umfasst die Anordnung zur Flüssigkeitsdetektion eine Backsheetfolie mit einem leitfähigen Aufdruck, der einen flächenspezifischen Widerstand bzw. einen spezifischen Flächenwiderstand von weniger als 10 kΩ aufweist. In einer besonders vorteilhaften Variante der Erfindung beträgt der flächenspezifische Widerstand des leitfähigen Aufdrucks weniger als 6 kΩ, vorzugsweise weniger als 4 kΩ, insbesondere weniger als 1 kΩ.

Bei einer vorteilhaften Variante der Erfindung beträgt das Flächengewicht des leitfähigen Aufdrucks weniger als 3 g/m², bevorzugt weniger als 2 g/m², vorzugsweise weniger als 1 g/m², insbesondere weniger als 0,5 g/m². Erfolgt beispielsweise ein Nassauftrag von 2 * 3,5 g an leitfähigen Medium pro m² bei einer Bedeckung von 11 % und einem Feststoffanteil des leitfähigen Medium von 35 %, so ergibt sich eine Flächengewicht des trockenen leitfähigen Aufdrucks von 7 g/m² * 0,11 * 0,35 = 0,2695 g/m².

Der flächenspezifische Widerstand wird von der Auftragsmenge einer leitfähigen Druckfarbe, der Dicke des Aufdrucks und einer Art der Behandlung konfiguriert. Der flächenspezifische Widerstand hat direkt nach dem Aufdrucken seinen geringsten Wert. Dabei hängt eine Veränderung dieses flächenspezifischen Widerstands entscheidend von den Eigenschaften, insbesondere von der Steifigkeit bei gleichzeitiger Elastizität, der Backsheetfolie ab. Nur durch die erfindungsgemäße Backsheetfolie, die besonders steif und dabei gleichzeitig elastisch ausgebildet ist, bleibt der aufgedruckte, flächenspezifische Widerstand auch bei der weiteren Verarbeitung möglichst gering. Der Einsatz spezifischer, polyolefinischer Backsheetfolien gewährleistet, dass die Leiterbahnen des Aufdrucks nicht während der weiteren Verarbeitungsschritte, beispielsweise durch Risse, beschädigt werden.

Der flächenspezifische Widerstand des Aufdrucks kann mit einem Multimeter über zwei Messspitzen erfasst werden.

Der flächenspezifische Widerstand des Aufdrucks kann mit Hilfe der Vier-Punkt-Methode oder als berührungslose Messung mit einem speziellen Wirbelstromprüfgerät erfasst werden. Oft wird anhand einer bekannten Geometrie zurückgerechnet. Bei der Vier-Punkt-Methode wird der Einfluss des Kontaktwiderstandes auf die Messung eliminiert, indem zwischen zwei Kontaktpunkten ein Stromfluss erzeugt wird, während der Spannungsabfall über zwei weitere Kontaktpunkte gemessen wird. Bei der berührungsfreien Schichtwiderstandsmessung mit Wirbelstrom wird im Material ein elektromagnetisches Wechselfeld erzeugt, dessen Gegenfeld der Messsensor auswertet.

Der spezifische Flächenwiderstand *R*_{□} beschreibt den elektrischen Widerstand einer elektrisch leitfähigen Schicht einer so geringen Dicke, dass diese lediglich parallel zur Schicht von elektrischem Strom durchflossen wird, d. h. der Strom tritt an einer Stirnfläche ein und an der gegenüberliegenden Stirnfläche wieder aus.

Vorzugsweise wird der flächenspezifische Widerstand *R*_{□} einer Backsheetfolie an einem aufgedruckten Streifen des leitfähigen Aufdrucks gemessen. Beispielsweise hat der Streifen eine Länge von 30 cm und eine Breite von 6 mm, was in diesem Fall 50 □ entspricht, wobei sich aus einem gemessenen Widerstand von beispielsweise 60 kΩ ein flächenspezifischer Widerstand von 1,2 kΩ/□ ergeben würde.

Zur besseren Unterscheidung vom elektrischen Widerstand mit der Einheit Ω wird der spezifische Flächenwiderstand *R*_{□} deshalb oft in der Einheit Ω/□ angegeben. Eine solche Indizierung physikalischer Einheiten ist jedoch nicht in den Normen DIN 1301 und ISO 31 vorgesehen, weshalb in den Ansprüchen die Einheit Ω für den spezifischen Flächenwiderstand *R*_{□} angeführt wird.

Der Aufdruck kann als für eine Flüssigkeitsdetektion besonders geeignetes Druckmuster ausgeführt sein. Das Druckmuster ist im einfachsten Fall ein Streifen. Alternativ kann es in Form mehrerer Streifen ausgebildet sein, die sich über die Anordnung erstrecken. Darüber hinaus kann das Druckmotiv auch spiralförmig, dreiecksförmig, rechteckig und/oder in einer völlig zufälligen geometrischen Gliederung ausgeführt sein. Bei einer Variante sind die Leiterbahnen schachbrettartig ausgebildet.

Die für den leitfähigen Aufdruck einzusetzenden Druckfarben sind vorzugsweise Druckfarben mit einer geringen Viskosität und somit nahezu wasserdünn. Prinzipiell eignen sich wässrige, UV-härtbare und lösemittelhaltige Farbsysteme. Diese Druckfarben können speziell an das jeweiligen Druckverfahren angepasst und werden daher individuell an die jeweilige Druckmaschine angepasst.

Leitfähige Druckfarben können insbesondere durch Wärme und/oder UV-Licht getrocknet bzw. ausgehärtet werden. Leitfähige Druckfarben enthalten in der Regel Partikel von leitfähigen Materialien, wie beispielsweise Graphit, Kupfer oder Silber, wobei die Partikel in Flocken- oder Pulverform vorgesehen sein können.

Im Rahmen der Erfindung wird vorzugsweise eine leitfähige Druckfarbe auf der Basis von Graphit bevorzugt. Vorzugsweise ist der leitfähige Aufdruck auf Basis einer kohlenstoffbasierten Tinte und/oder einer leitenden, polymerbasierten Tinte ausgeführt. Die kohlenstoffbasierte Tinte umfasst vorzugweise eine leitende Verbindung, die aus der Gruppe bestehend aus Graphen, Graphit, Nanokohlenstoffröhrchen und Mischungen davon gebildet ist. Eine leitende polymerbasierte Tinte umfasst vorzugsweise eine leitende Verbindung, die aus der Gruppe aus Polyacetylen, Polypyrrol, Polyanilin und deren Copolymeren besteht. Insbesondere kann die leitfähige Tine aus Polypyrrolen (PPY), Polyanilinen (PANI), Polythiophenen (PT), Polyphenylensulfid (PPS), Polyphenylen (PPP), Polyacetylenen (PAC), Polyphenylenvinylen (PPV), Poly(3,4-ethylendioxythiophen) (PEDOT) und deren Mischungen bestehen, wobei die leitende polymerbasierte Tinte am besten ein Poly(3,4-ethylen-dioxythiophen)-Polystyrolsulfonat (PEDOT:PSS) umfasst.

Vorzugsweise wird der leitfähige Aufdruck mit einem Flexodruck-Verfahren aufgebracht, wobei alle gängigen Druckverfahren prinzipiell dafür geeignet und ausdrücklich in die Erfindung mit eingeschlossen sind.

Vorteilhafterweise weist der leitfähige Aufdruck auf der Backsheetfolie keinen Abrieb gemäß dem Ink Rub-off-test auf. Dadurch ist unabhängig von der Anordnung des Aufdrucks beim Tragen des Hygieneartikels ein Übertragen des Aufdrucks auf die Haut des Hygieneartikeltragenden auszuschließen.

Beim Ink Rub-off-test wird ein Prüfarm mit stoffartigem Material auf die bedruckte Backsheetfolie gelegt. Dann pendelt der Tisch unter der Backsheetfolie 15-mal hin und her. Anschließend wird die Farbübertragung auf das stoffartige Material bewertet, wobei bestanden ohne Farbabrieb, bestanden mit leichtem Farbabrieb und nicht bestanden die drei Bewertungskategorien darstellen.

Die erfindungsgemäße Anordnung weist eine spezifische Kombination von Merkmalen auf, die bei herkömmlichen Anordnungen zur Flüssigkeitsdetektion gemäß dem Stand der Technik nicht bekannt sind. Durch diese spezifische Kombination an Merkmalen ist es möglich, auch dünne und besonders einfache Anordnungen einzusetzen, die einen leitfähigen Aufdruck aufweisen.

Die erfindungsgemäße Anordnung weist gegenüber herkömmlichen Anordnungen für intelligente Windeln deutlich bessere mechanischen Eigenschaften auf, ist besonders soft sowie sanft und kommt dabei ganz ohne komplizierte elektrische Verschaltungen und deren komplexen Aufbauten aus. Die erfindungsgemäße Anordnung ist frei von Drähten, elektrischen Leiterbahnen, Widerstandsschaltungen und deren isolierenden zusätzlichen Schichten.

Die erfindungsgemäße Anordnung ist besonders dünn und gleichzeitig steif, so dass der Aufdruck in der Verarbeitung in modernen Windelkonverter keinen Schaden nimmt. Zudem sichert die erfindungsgemäße Anordnung dennoch zuverlässig eine Durchnässung trotz dessen einfachen Aufbau aus einer Backsheetfolie, einem leitfähigen Aufdruck und einem Vlies.

Bei einer Variante der Erfindung umfasst die Anordnung eine Vlieslage. Der Ausdruck "Vlies" bezieht sich auf einen Stoff, der aus kontinuierlichen Filamenten und/oder diskontinuierlichen Fasern ohne Weben oder Stricken mittels Verfahren wie Spunbonding, Kardieren oder Meltblowing hergestellt werden kann. Der Vliesstoff kann eine oder mehrere Lagen Vlies umfassen, wobei jede Lage kontinuierliche Filamente oder diskontinuierliche Fasern enthalten kann. Vlies kann auch Bikomponentenfasern umfassen, welche Faserstrukturen wie zum Beispiel Mantel/Kern-, Seit-an-Seit aufweisen können.

Vorzugsweise besteht die Vlieslage aus einem polyolefinischen Vlies, insbesondere einem thermobondierten Spinnvlies. Dabei eignet sich insbesondere ein polypropylenbasierter Vliesstoff. Die Vlieslage weist vorzugsweise ein spezifisches Gewicht von mehr als 6 g/m², vorzugsweise von mehr als 8 g/m², insbesondere von mehr als 10 g/m² und/oder weniger als 20 g/m², vorzugsweise weniger als 18 g/m², insbesondere weniger als 16 g/m² auf.

Zwischen der Vlieslage und der Backsheetfolie sind Verbindungsbereiche angeordnet. Vorteilhafterweise sind die Verbindungsbereiche als formschlüssiger Verbund aus Vlies und erstarrtem Material der Backsheetfolie ausgebildet. Dies wird erreicht, in dem in einem Verfahren zur Herstellung einer Anordnung aus einer Backsheetfolie und einer Vlieslage das thermoplastischen Polymermaterial der Backsheetsfolie über den Kristallitschmelzpunkt des Polymermaterials hinaus erwärmt und anschließend zusammen mit der Vlieslage durch einen gekühlten Walzenspalt geführt wird.

In einer besonders vorteilhaften Variante der Erfindung ist der leitfähige Aufdruck auf der Seite der Backsheetfolie angeordnet, auf der die Vlieslage mittels Thermolaminierung verbunden wird. Dies ist nur möglich, weil die Backsheetfolie besonders steif ausgebildet ist und der leitfähige Aufdruck in der Weiterverarbeitung dadurch nicht reißen kann.

Als besonders günstig erweist es sich, wenn der Aufdruck unmittelbar auf der Backsheetfolie angeordnet ist. So kann beispielsweise auf das Aufbringen eines Haftvermittlers verzichtet werden, wodurch auf einen zusätzlichen Prozessschritt verzichtet werden kann. Gleichzeitig lässt sich durch das unmittelbare Aufbringen des Aufdrucks die Anordnung besonders dünn ausbilden. Weiterhin müssen keine zusätzlichen Elemente eingebracht werden, beispielsweise zur Detektion einer Flüssigkeit. Dies ist insbesondere vorteilhaft, da keine potentielle Fehlerquellen durch das Anbringen zusätzlicher Elemente und durch das Ausführen weiterer Arbeitsschritte wie das Abrollen der Folienbahn sowie das Anbringen der Elemente entstehen.

Die Anordnung wird vorzugsweise in einem Verfahren hergestellt, bei dem eine spezifische Zusammensetzung zunächst zu einer Folienbahn extrudiert wird, vorzugsweise mittels Blasextrusion und zur weiteren Verarbeitung gekühlt wird. Die Folienbahn wird dann in Maschinenrichtung und/oder quer zur Maschinenrichtung gereckt. Erfindungsgemäß erfolgt das Aufbringen des leitfähigen Aufdrucks in einem Inline-Prozess. Bei dem Inline-Prozess muss die Folienbahn vor dem Verbinden mit einer Vlieslage nicht aufgerollt und erneut wieder abgeholt werden. Das Aufbringen der Vlieslage erfolgt "inline" und somit vorzugsweise unmittelbar nach dem Aufbringen des leitfähigen Ausdrucks in einem Prozess. Zum Abschluss des Prozesses kann das Vlies mobilisiert werden. Dies geschieht vorzugsweise mittels eines Ringrollings. Dadurch wird die Softness-Anmutung der Anordnung gesteigert.

Idealerweise ist der Aufdruck zwischen der Backsheetfolie und der Vlieslage angeordnet. Das erfindungsgemäße Inline-Verfahren erlaubt einen formschlüssigen Verbund zwischen Backsheetfolie und Vlies auszubilden, ohne den Aufdruck in seiner Leitfähigkeit negativ zu beeinflussen.

Das Verfahren (nicht beansprucht) ist besonders vorteilhaft, weil die fertige Anordnung auf Bahnen aufgerollt werden kann, ohne dass zuvor die Folienbahn nach dem Aufbringen des leitfähigen Aufdrucks separat aufgerollt werden muss, wobei letzterer durch erneute Spannungseinwirkung geschädigt und seine Leitfähigkeit reduziert werden könnte.

Die Backsheetfolie umfasst vorzugsweise thermoplastische Anteile, wobei es sich als besonders günstig erweist, wenn die Polymermaterialien von Backsheetfolie und Vlieslage so aufeinander abgestimmt sind, dass einerseits die Kristallitschmelzpunkte weit genug auseinander liegen und andererseits die Materialien soweit miteinander verträglich sind, dass eine Verbindung ermöglicht ist.

Der Unterschied in der Kristallitschmelztemperatur der niedrigschmelzenden Komponente der Backsheetfolie sollte mindestens etwa 5 °C, vorzugsweise mindestens etwa 10 °C und insbesondere mindestens etwa 20 °C unterhalb der Schmelztemperatur des Vlieses bzw. unterhalb der Schmelztemperatur der hochschmelzenden Komponente des Vlieses liegen.

Erfindungsgemäß umfasst die Backsheetfolie vorzugsweise wenigstens eine niedrig schmelzende Polymerkomponente und wenigstens eine hoch schmelzende Polymerkomponente.

Die Gesamtmenge an niedrig schmelzender Polymerkomponente beträgt vorzugsweise 90 bis 30 Gew.-%, insbesondere 80 bis 40 Gew.-%, am meisten bevorzugt 70 bis 50 Gew.-%, die Gesamtmenge an hoch schmelzender Polymerkomponente beträgt vorzugsweise 10 bis 70 Gew.-%, insbesondere 20 bis 60 Gew.-%, am meisten bevorzugt 30 bis 50 Gew.-%, jeweils bezogen auf 100 Gew.-% niedrig schmelzende und hoch schmelzende Polymerkomponente.

In einer Ausführungsform enthält die Backsheetfolie wenigstens ein Polyethylen als niedrig schmelzende Polymerkomponente und wenigstens ein Polypropylen als hoch schmelzende Polymerkomponente.

Bei einer Variante enthält die niedrig schmelzende Polymerkomponente Ethylenpolymere oder besteht aus diesen, wobei sowohl Ethylenhomopolymere als auch Ethylencopolymere mit Ethylen als Hauptmonomerem sowie Gemische (Blends) von Ethylenhomopolymeren und Ethylencopolymeren geeignet sind. Geeignete Ethylenhomopolymere sind LDPE (Low Density Polyethylene), LLDPE (Linear Low Density Polyethylene), MDPE (Medium Density Polyethylene) und HDPE (High Density Polyethylene).

In einer Ausführungsform besteht die niedrig schmelzende Polymerkomponente ausschließlich aus Ethylenhomopolymeren, z. B. Gemischen aus LDPE und LLDPE, die jeweils in Mengen von 10 bis 90 Gew.-% enthalten sein können, sowie 0 bis 50 Gew.-% MDPE.

Vorzugsweise enthält die hoch schmelzende Polymerkomponente wenigstens ein Polypropylen, dessen Schmelzpunkt, Schmelzbereich oder Kristallitschmelzpunkt wesentlich höher als der der niedrig schmelzenden Polymerkomponente liegt. Geeignetes Polypropylen ist insbesondere isotaktisches Polypropylen. Es kann auch syndiotaktisches Polypropylen verwendet werden, sofern dessen Schmelzpunkt, Schmelzbereich oder Kristallitschmelzpunkt wesentlich höher als der der niedrig schmelzenden Polymerkomponente liegt.

Die hoch schmelzende Polymerkomponente kann sowohl Propylenhomopolymere als auch Propylencopolymere mit Propylen als Hauptmonomerem umfassen.

Vorzugsweise weisen die zu verbindenden Backsheetfolienbahnen und Vlieslagen zumindest in einer Rezepturkomponente eine ähnliche Morphologie auf.

Die Backsheetfolie wird erfindungsgemäß gemeinsam mit der Vlieslage über einen vorzugsweise antihaft-beschichten Heizzylinder erwärmt und anschließend durch einen gekühlten Walzenspalt geführt. Es kann natürlich auch mit mehreren Heizzylindern oder anderen Erwärmungsmethoden wie z.B. Infrarotstrahler gearbeitet werden.

In einer bevorzugten Ausführungsform steht die Vlieslage im direkten Kontakt mit der Heizzylinderoberfläche. Darüber wird die Backsheetfolie mit dem leitfähigen Aufdruck mitgeführt. Die Temperatur des Heizzylinders wird so gewählt, dass über die Umschlingungsstrecke des Heizzylinders eine Komponente der Backsheetfolie in den schmelzeflüssigen Zustand erwärmt wird, ohne den leitfähigen Aufdruck zu beeinträchtigen. Diese Temperatur versetzt das Vlies noch nicht in den schmelzeflüssigen Zustand.

Da das noch nicht im schmelzeflüssigen Zustand befindliche Vlies auf dem Heizzylinder aufliegt ist ein leichtes und sehr prozessstabiles Ablösen der Backsheetfolie gewährleistet.

Im anschließenden gekühlten Walzenspalt wird der Vlies-Folien-Verbund auf Temperaturen unterhalb des Kristallitschmelzpunkts der Backsheetfolie gekühlt. Der gekühlte Walzenspalt besteht bevorzugt aus einer Stahlwalze und einer im Gegendruck arbeitenden Gummiwalze.

Im Gegensatz zu bekannten Thermobonding-Laminierverfahren, bei dem durch zwei beheizte Stahlwalzen mittels Temperatur und sehr hoher Drücke der Verbund nur punktuell geschaffen wird, erzeugt das Thermolaminier-Verfahren Verbindungsbereiche mit einer vollflächigen Laminierung. Dies bietet, ähnlich den bekannten Kleberlaminierverfahren, den Vorteil, dass durch geringe Drücke im Laminierprozess sehr weiche Anordnungen ohne Kleber geschaffen werden. Des Weiteren besteht im Gegensatz zu Thermobondinglaminaten keine Gefahr von Materialschädigungen (Löcher, Pinholes), was gerade hinsichtlich des leitfähigen Aufdrucks enorm wichtig ist.

In einer alternativen Variante der Erfindung ist es vorstellbar, dass die Anordnung aus Vlieslage und Backsheetfolie über einem Hotmelt-Kleber verbunden wird. Auch ein Verbinden der Folienbahn mit einer Vlieslage über ein Ultraschall-Verschweißen ist denkbar.

Die Folie kann nach der Extrusion und vor dem Laminieren bedruckt werden. Dies ermöglicht den Druck auf die Backsheetfolienseite, die in der späteren Anordnung mit der Vlieslage abgedeckt wird. Damit erreicht man eine sehr gute Druckqualität, weil auf die glatte Backsheetfolie gedruckt werden kann. Dadurch kann der leitfähige Aufdruck zur Flüssigkeitsdetektion herangezogen werden und gleichzeitig verschließt die Backsheetfolie die Anordnung flüssigkeitsdicht. Zudem schützt die verbundene Vlieslage den leitfähigen Aufdruck in der weiteren Verarbeitung zum Einweghygieneartikel.

Vorzugsweise ist das Bedrucken in den Produktionsprozess inline integriert. An die Extrusion der Backsheetfolie schließt sich vorzugsweise ein Prägewerk, und/oder ein Chili-Roll System und/oder mindestens eine Kühlwalze an.

Bei einer Variante wird das Vlies-Folienlaminat einem Ringrolling unterzogen. Dieser sanfte Prozessschritt mobilisiert die Fasern und erhöht die Weichheit sowie die Softness des Laminats. Diese beschriebenen Eigenschaftsveränderungen können leicht durch die verwendete Geometrie und den Eingriffsgrad der Ringrollingwalzen beeinflusst werden. Nur die erfindungsgemäßen Eigenschaften der Backsheetfolie ermöglichen eine sanfte Behandlung mit dem Ringrolling, ohne dass der flächenspezifische Widerstand des leitfähigen Aufdrucks maßgeblich verändert wird.

Bei einer Variante der Erfindung ist die Backsheetfolie nicht atmungsaktiv ausgebildet und weist eine Wasserdampfdurchlässigkeit von weniger als 500 g/m² in 24 h nach ASTM D6701-01 auf. Das Flächengewicht der nicht atmungsaktiven Backsheetfolie beträgt vorzugsweise weniger als 10 g/m², vorzugsweise weniger als 8 g/m², insbesondere weniger als 6 g/m².

Dabei beträgt der spezifische Dart-Drop nach ASTM D1709A der Anordnung vorzugsweise mehr als 6 g je Gramm Polymer pro Quadratmeter, so dass sich der Wert aus der Masse des "Darts" geteilt durch das spezifische Gewicht des Folien-Vlies-Laminats also zu 6 g / (g/m²) ergibt und/oder die spez. Wassersäule der Anordnung mehr als 270 mm je Gramm Polymer pro Quadratmeter, so dass sich der Wert aus der Wassersäule geteilt durch das spezifische Gewicht des Folien-Vlies-Laminats also zu 270 mm / (g/m²) ergibt.

Diese vorteilhaften Eigenschaften der erfindungsgemäßen Anordnung zeigen sich anhand charakteristischer Kennwerte. Die Anordnung weist eine Kraft von mindestens 0,3 N/in, vorzugsweise eine Kraft von mindestens 0,375 N/in, insbesondere eine Kraft von mehr als 0,45 N/, jeweils bei 5 % Dehnung je Gramm Polymer pro Quadratmeter auf, so dass sich der Wert aus der Kraft pro Inch geteilt durch das spezifische Gewicht des Folien-Vlies-Laminats also ergibt.

Wird beispielsweise ein Wert von 8,5 N/in gemessen, bei einem Folien-Vlies-Laminats mit einem spezifischen Gewicht von 18 g/m², so ergibt sich ein Wert von 0,47222 (N/in) / (g/m²).

Die Messung der Kraft bei 5 % Dehnung ist nach der ASTM D882 erfolgt. Dadurch ist die nicht atmungsaktive Backsheetfolie besonders steif ausgebildet, wodurch ein leitfähiger Aufdruck besonders geschützt vor einem Reißen des Aufdrucks ist. Aufgrund der erfindungsgemäßen Rezeptur ist die Backsheetfolie zudem sehr elastisch, was zum Schutz des leitfähigen Aufdrucks und zur Erzeugung einer angenehmen Haptik vorteilhaft beträgt.

In einer weiteren Variante der Erfindung ist die Backsheetfolie atmungsaktiv ausgebildet. Die atmungsaktive Backsheetfolie weist eine Wasserdampfdurchlässigkeit von mehr als 500 g/m² in 24 h nach ASTM D6701-01 auf. Das Flächengewicht der atmungsaktiven Backsheetfolie beträgt vorzugsweise weniger als 20 g/m², bevorzugt weniger als 16 g/m², insbesondere weniger als 12 g/m².

Der spezifische Dart-Drop nach ASTM D1709A der atmungsaktiven Backsheetfolie beträgt vorzugsweise mehr als 26 g je Gramm Polymer pro Quadratmeter und/oder die spez. Wassersäule der atmungsaktiven Backsheetfolie beträgt mehr als 460 mm je Gramm Polymer pro Quadratmeter.

Die besonderen mechanischen Eigenschaften der atmungsaktiven Backsheetfolie sind in einer spezifischen Rezeptur in Kombination mit einer ganz spezifischen Verarbeitung begründet, wobei bei der Herstellung der Folie vorzugsweise eine Blasextrusion zum Einsatz kommt.

Die Backsheetfolie weist bei einer Variante der Erfindung ein niedrig schmelzendes Polypropylen und ein hoch schmelzendes Polypropylen auf.

Dabei erweist es sich als besonders, wenn der Unterschied im Kristallitschmelzpunkt zwischen dem niedrig schmelzenden Polypropylen und dem hoch schmelzenden Polypropylen in der Backsheetfolie mehr als 10 °C, vorzugsweise mehr als 15 °C, insbesondere mehr als 20 °C beträgt und/oder weniger als 50 °C, vorzugsweise weniger als 40 °C, insbesondere weniger als 30 °C beträgt.

Bei einer Variante der Erfindung weist die Zusammensetzung vorzugsweise CaCO₃ mit einem Anteil von 40 bis 60 Gew.-% auf. In einer besonders vorteilhaften Variante der Erfindung weist die Zusammensetzung der atmungsaktiven Backsheetfolie ein LDPE mit einem Anteil von mehr als 2 Gew._% und/oder weniger als 10 Gew.-% auf.

Die atmungsaktive Backsheetfolie weist vorzugsweise eine Kraft von mindestens 0,5 N/in, bevorzugt eine Kraft von mehr als 0,6 N/in, insbesondere eine Kraft von mehr als 0,7 N/in pro Gramm Polymer pro m² jeweils bei 5 % Dehnung auf. Die Messung der Kraft bei 5 % Dehnung ist nach der ASTM D882 erfolgt. Die atmungsaktive Backsheetfolie ist sehr steif ausgebildet, wodurch der leitfähige Aufdruck einen Schutz vor möglichen Beschädigungen erfährt. Darüber hinaus ist Backsheetfolie sehr elastisch ausgebildet, wodurch eine angenehme Haptik erzeugt wird.

Wird beispielsweise ein Wert von 4,2 N/in bei 5% Dehnung gemessen, bei einer Folie mit einem spezifischen Gewicht von 16 g/m² und einem Polymeranteil von 40 %, so ergibt sich ein Wert von (4,2 N/in / 16 g/m²) / 0,4 = 0,65625 (N/in) / (g/m²).

Somit zeichnet sich die erfindungsgemäße Anordnung als besonders leicht aus, wodurch ein angenehmes Tragegefühl erzeugt werden kann.

Diese günstige Steifigkeit in Kombination einer vorteilhaften Softness der Anordnung wird durch eine spezifische Zusammensetzung, einer gezielten Auswahl an Polymeren in Kombination mit einem spezifischen Herstellungsprozess erreicht.

Die erfindungsgemäße Anordnung zur Flüssigkeitsdetektion wird vorzugsweise im Hygiene- oder Medikalbereich, insbesondere für Einweghygieneartikel verwendet. Dabei können die Einweghygieneartikel sowohl bei den jüngsten als auch bei den ältesten unserer Gesellschaft Anwendung finden.

An dem Einweghygieneartikel kann ein Auslesen des flächenspezifischen Widerstands mit einem Lesegerät erfolgen, welches an den Einweghygieneartikel angeclipst werden kann und mit dem leitfähigen Druckmuster eine Detektionseinheit bildet. Dadurch wird der Einweghygieneartikel zur intelligenten Windel. Wenn eine angrenzende Flüssigkeit leitend mit dem leitfähigen Druckmuster verbunden ist, so ist ein deutliche Widerstandsänderung mit dem Lesegerät feststellbar. Das Lesegerät weist vorzugsweise eine Bluetooth-Sende/Empfangseinheit auf, wodurch eine raumungebundene Übermittlung und Anzeige eines Feuchtigkeitsereignisses ermöglicht werden kann.

### Beispiel 1: Anordnung zur Flüssigkeitsdetektion mit einer atmungsaktiven Backsheetfolie

Bei diesem Beispiel kommen folgende Komponenten zur Herstellung der Backsheetfolie zum Einsatz:

| Menge Gew.-% | Bestandteil | Dichte, g/cm³ | Kristallitschmelzpunkt, °C |
|---|---|---|---|
| 32 | PP | 0,9 | 140-142 |
| 58 | CaCO₃ | - | |
| 6 | PP | 0,9 | 163-167 |
| 4 | LDPE | 0,92 | 111 |

Bei dem eingesetzten Füllstoff handelt es sich um einen anorganischen Füllstoff in Form von Calciumcarbonat, vorzugsweise mit einer Partikelgröße von 0,8 bis 2 µm.

Zur Herstellung der erfindungsgemäßen Backsheetfolie werden die Polymerbestandteile mit den mineralischen Füllstoffen in einem Extruder, zum Beispiel einem Compounding-Extruder auf eine Temperatur deutlich über der Schmelztemperatur der Polymerbestandteile erhitzt (zum Beispiel über 200°C) und miteinander verschmolzen.

Dann schließt sich erfindungsgemäß eine Blasextrusion mit Kühlung der Folienbahn an. Im anschließenden monoaxialen Reckprozess wird die Backsheetfolie vorzugsweise 100 % in Maschinenrichtung verstreckt.

Im Inline-Verfahren folgt der Aufdruck mit leitfähiger Druckfarbe im Flexodruckverfahren unmittelbar auf die atmungsaktive Backsheetfolie. Die leitfähige Druckfarbe enthält vorzugsweise Graphitpigmente, die in einem Gemisch aus Propylacetat und Propan-2-ol gelöst sind. Vorzugsweise weist die leitfähige, schwarze Druckfarbe Füllstoffe wie Cellulosenitrat auf.

Die Backsheetfolie wird folgend mit einer auf Polypropylen basierten, thermobondierten Vlieslage, die ein spezifisches Flächengewicht von 14 g/m² aufweist, über einen Heizzylinderoberfläche geführt, so dass die Backsheetfolie teilweise im geschmolzenen Zustand befindet und im anschließenden gekühlten Walzenspalt flächige Verbindungsbereiche mit der Vlieslage bildet. Der leitfähige Aufdruck auf der atmungsaktiven Backsheetfolie ist durch die verbundene Vlieslage abgedeckt. In einem abschließenden Ringrolling werden die Fasern des Folien-Vlies-Verbundes schonend aktiviert, wodurch eine ansprechende weiche Haptik erzielt wird.

Der flächenspezifische Widerstand des leitfähigen Aufdrucks wird anhand eines Teststreifens mit einer Länge von 30 cm und eine Breite von 6 mm bestimmt, was 50 Quadrate entspricht. Dabei werden die Messspitzen eines Multimeters an den Enden aufgedrückt und ein Widerstandswert von beispielsweise 57 kΩ gemessen. Daraus ergibt sich rechnerisch ein flächenspezifischer Widerstand von 1,14 kΩ/Quadrate.

### Beispiel 2: Anordnung zur Flüssigkeitsdetektion mit einer nicht atmungsaktiven Backsheetfolie

Bei diesem Beispiel kommen folgende Komponenten zum Einsatz:

| Menge Gew.-% | Bestandteil | Dichte, g/cm³ | Kristallitschmelzpunkt, °C |
|---|---|---|---|
| 37,5 | LDPE | 0,92 | 111 |
| 25,5 | LLDPE | 0,92 | 123 |
| 16,25 | PP | 0,90 | 163 - 167 |
| 7,5 | PP | 0,90 | 140 - 142 |
| 8,0 | TiO₂ | - | |
| 4,5 | CaCO₃ | - | |
| 0,5 | Thermostabilisator | 0,93 | |
| 0,25 | Hilfsmittel | 0,94 | |

Zur Herstellung der erfindungsgemäßen Folienbahn werden die Polymerbestandteile in einem Extruder, zum Beispiel einem Compounding-Extruder auf eine Temperatur deutlich über der Schmelztemperatur der Polymerbestandteile erhitzt und miteinander verschmolzen. Anschließend erfolgt die Blasextrusion mit Kühlung der mehrschichtigen Folienbahn an. Im erfindungsgemäßen Inline-Verfahren wird der Aufdruck mit leitfähiger Druckfarbe im Flexodruckverfahren unmittelbar auf die nicht atmungsaktive Backsheetfolie aufgetragen. Ein alternative, leitfähige Druckfarbe enthält typischerweise Graphitpigmente, die in einem Gemisch aus Wasser und Ammoniumhydroxid gelöst sind.

Die nicht atmungsaktive Backsheetfolie wird folgend mit einer auf Polypropylen basierten, thermobondierten Vlieslage, die ein spezifisches Flächengewicht von 14 g/m² aufweist, über einen Heizzylinderoberfläche geführt, so dass die Backsheetfolie teilweise im geschmolzenen Zustand befindet und im anschließenden gekühlten Walzenspalt flächige Verbindungsbereiche mit der Vlieslage bilden. In einem abschließenden Ringrolling werden die Fasern des Folien-Vlies-Verbundes schonend aktiviert, wodurch eine ansprechende weiche Haptik erzielt wird.

Der flächenspezifische Widerstand des leitfähigen Aufdrucks wird anhand eines Teststreifens mit einer Länge von 30 cm und eine Breite von 6 mm bestimmt, was 50 _{□} entspricht. Dabei werden die Messspitzen eines Multimeters an den Enden aufgedrückt und ein Widerstandswert von beispielsweise 56 kΩ gemessen. Daraus ergibt sich rechnerisch ein spezifischer Flächenwiderstand von 1,12 kΩ/□.

Weitere Vorteile und Merkmale der Erfindung ergeben sich aus der Beschreibung verschiedener Ausführungsbeispiele anhand von Zeichnungen und aus den Zeichnungen selbst.

Dabei zeigt
Fig. 1 eine schematische Darstellung einer Backsheetfolie mit einem Aufdruck,
Fig. 2 eine schematische Darstellung der erfindungsgemäßen Anordnung,
Fig. 3 eine weitere Variante der erfindungsgemäßen Anordnung,
Fig. 4 eine dritte Variante der erfindungsgemäßen Anordnung.

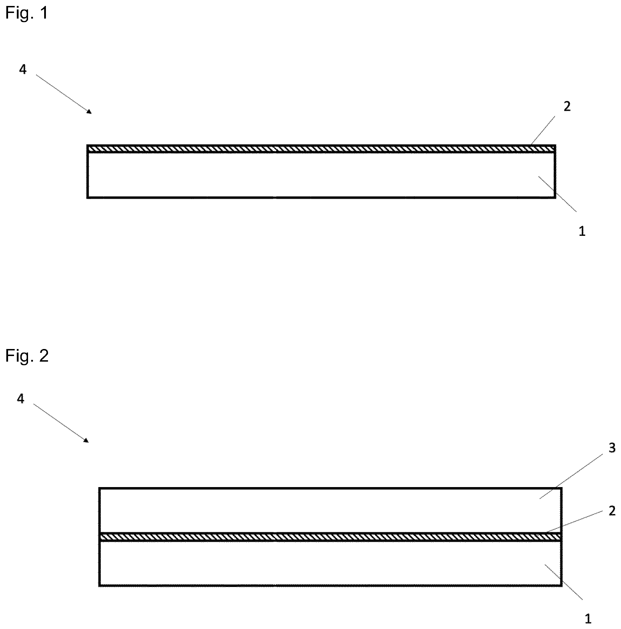

In Fig. 1 ist eine Backsheetfolie 1 mit einem leitfähigen Aufdruck 2 dargestellt, wobei der leitfähige Aufdruck 2 direkt und ohne Haftvermittlerschicht auf der Backsheetfolie 1 angeordnet ist. Die Backsheetfolie 1 ist atmungsaktiv ausgeführt und weist ein spezifisches Flächengewicht von 12 m²/g auf. Das Flächengewicht des leitfähigen Aufdrucks beträgt weniger als 0,5 g/m².

Fig. 2 zeigt eine Anordnung gemäß Fig. 1, wobei zusätzlich eine Vlieslage 3 mit der Backsheetfolie 1 verbunden ist. Die Vlieslage 3 weist ein spezifisches Flächengewicht von 12 g/m².

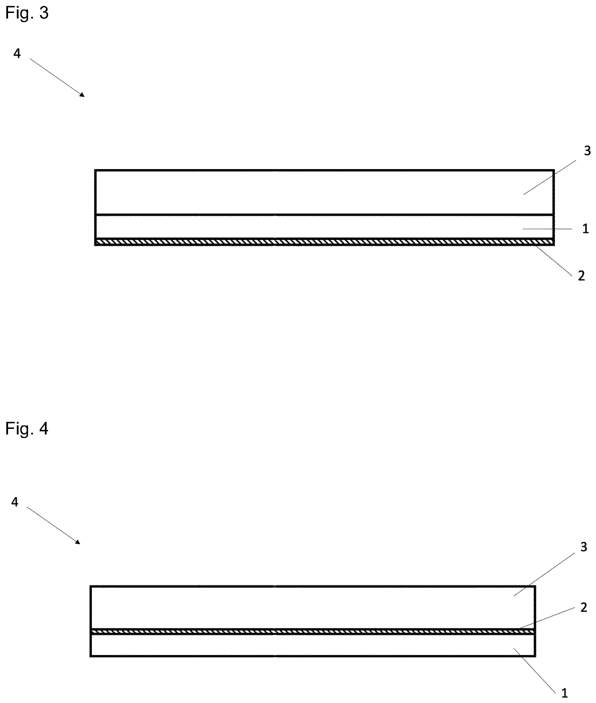

In Fig. 3 ist eine Anordnung dargestellt, bei der die Backsheetfolie 1 mit einem leitfähigen Aufdruck 2 versehen ist, wobei die Vlieslage 3 auf der gegenüberliegenden Seite des leitfähigen Aufdrucks 2 angeordnet ist.

Fig. 4 zeigt eine Ausführung gemäß Fig.2, bei der die Backsheetfolie 1 nicht atmungsaktiv ausgeführt ist und ein Flächengewicht von 6 g/m² aufweist.

## Patentansprüche

1. Anordnung (4) zur Flüssigkeitsdetektion in Einweghygieneartikel, **dadurch gekennzeichnet, dass** die Anordnung (4) eine Backsheetfolie (1) mit einem leitfähigen Aufdruck (2) umfasst, wobei das Flächengewicht des leitfähigen Aufdrucks (2) weniger als 3 g/m² beträgt, wobei die Backsheetfolie (1) nicht atmungsaktiv ausgebildet ist und eine Wasserdampfdurchlässigkeit von weniger als 500 g/m² in 24 h nach ASTM D6701-01 aufweist, wobei das Flächengewicht der nicht atmungsaktiven Backsheetfolie (1) weniger als 10 g/m² beträgt oder die Backsheetfolie (1) atmungsaktiv ausgebildet ist und eine Wasserdampfdurchlässigkeit von mehr als 500 g/m² in 24 h nach ASTM D6701-01 aufweist, wobei das Flächengewicht der atmungsaktiven Backsheetfolie (1) weniger als 20 g/m² beträgt.

2. Anordnung nach Anspruch 1, **dadurch gekennzeichnet, dass** der leitfähige Aufdruck (2) einen flächenspezifischen Widerstand von weniger als 10 kΩ, bevorzugt weniger als 6 kΩ, vorzugsweise weniger als 4 kΩ, insbesondere weniger als 1 kΩ aufweist.

3. Anordnung nach Anspruch 1 oder 2, **dadurch gekennzeichnet, dass** das Flächengewicht des leitfähigen Aufdrucks (2) weniger als 2 g/m², vorzugsweise weniger als 1 g/m², insbesondere weniger als 0,5 g/m² beträgt.

4. Anordnung nach einem der Ansprüche 1 bis 3, **dadurch gekennzeichnet, dass** die mit dem leitfähigen Aufdruck (2) bedruckte Backsheetfolie (1) keinen Abrieb gemäß dem Rub-off-test aufweist.

5. Anordnung nach einem der Ansprüche 1 bis 4, **dadurch gekennzeichnet, dass** die Anordnung (4) eine Vlieslage (3) aufweist, wobei zwischen der Vlieslage (3) und der Backsheetfolie (1) Verbindungsbereiche angeordnet sind.

6. Anordnung nach Anspruch 5, **dadurch gekennzeichnet, dass** die Verbindungsbereiche als formschlüssiger Verbund aus Material der Vlieslage (3) und erstarrtem Material der Backsheetfolie (1) ausgebildet sind.

7. Anordnung nach einem der Ansprüche 1 bis 6, **dadurch gekennzeichnet, dass** der leitfähige Aufdruck (2) zwischen der Backsheetfolie (1) und der Vlieslage (3) angeordnet ist.

8. Anordnung nach einem der Ansprüche 1 bis 7, **dadurch gekennzeichnet, dass** die Backsheetfolie (1) nicht atmungsaktiv ausgebildet ist, wobei das Flächengewicht der Backsheetfolie (1) weniger als 8 g/m², insbesondere weniger als 6 g/m² beträgt.

9. Anordnung nach Anspruch 8, **dadurch gekennzeichnet,**
- **dass** der spezifische Dart-Drop der Anordnung (4) mehr als 6 g je Gramm Polymer pro Quadratmeter pro beträgt
und/oder
- die spez. Wassersäule der Anordnung (4) mehr als 270 mm je Gramm Polymer pro Quadratmeter beträgt.

10. Anordnung nach Anspruch 8 oder 9, **dadurch gekennzeichnet, dass** die Anordnung (4)
- eine Kraft von mindestens 0,3 N/in pro Gramm Polymer pro m²,
- vorzugsweise eine Kraft von mindestens 0,375 N/in pro Gramm Polymer pro m²,
- insbesondere eine Kraft von mehr als 0,45 N/in pro Gramm Polymer pro m²,
jeweils bei 5 % Dehnung aufweist.

11. Anordnung nach einem der Ansprüche 1 bis 7, **dadurch gekennzeichnet, dass** die Backsheetfolie (1) atmungsaktiv ausgebildet ist, wobei das Flächengewicht der Backsheetfolie (1) weniger als 16 g/m², insbesondere weniger als 12 g/m² beträgt.

12. Anordnung nach Anspruch 11, **dadurch gekennzeichnet, dass**
- der spezifische Dart-Drop der Backsheetfolie (1) mehr als 26 g je Gramm Polymer pro Quadratmeter beträgt und/oder
- die spez. Wassersäule der Backsheetfolie (1) mehr als 460 mm je Gramm Polymer pro Quadratmeter beträgt.

13. Anordnung nach Anspruch 11 oder 12, **dadurch gekennzeichnet, dass** die Backsheetfolie (1) eine Kraft von
- mindestens 0,5 N/in pro Gramm Polymer pro m²,
- vorzugsweise eine Kraft von mehr als 0,6 N/in pro Gramm Polymer pro m²,
- insbesondere eine Kraft von mehr als 0,7 N/in pro Gramm Polymer pro m²
jeweils bei 5 % Dehnung aufweist.

14. Anordnung nach einem der Ansprüche 1 bis 13, **dadurch gekennzeichnet, dass** der leitfähige Aufdruck (2) unmittelbar auf der Backsheetfolie (1) angeordnet ist.

## Claims

1. Arrangement (4) for liquid detection in disposable hygiene articles, **characterized in that** the arrangement (4) comprises a backsheet film (1) with a conductive print (2), wherein the basis weight of the conductive print (2) is less than 3 g/m² , wherein the backsheet film (1) is not designed to be breathable and has a water vapor transmission rate of less than 500 g/m² in 24 h according to ASTM D6701-01, wherein the basis weight of the non-breathable backsheet film (1) is less than 10 g/m² or the backsheet film (1) is designed to be breathable and has a water vapor transmission rate of more than 500 g/m² in 24 h according to ASTM D6701-01, wherein the basis weight of the breathable backsheet film (1) is less than 20 g/m².

2. Arrangement according to claim 1, **characterized in that** the conductive print (2) has a surface resistance of less than 10 kΩ, preferably less than 6 kΩ, preferably less than 4 kΩ, and in particular less than 1 kΩ.

3. Arrangement according to claim 1 or 2, **characterized in that** the basis weight of the conductive print (2) is less than 2 g/m², preferably less than 1 g/m², in particular less than 0.5 g/m².

4. Arrangement according to one of claims 1 to 3, **characterized in that** the backsheet film (1) printed with the conductive imprint (2) does not exhibit any abrasion according to the rub-off test.

5. Arrangement according to one of claims 1 to 4, **characterized in that** the arrangement (4) has a nonwoven layer (3), wherein connecting regions are arranged between the nonwoven layer (3) and the backsheet film (1).

6. Arrangement according to claim 5, **characterized in that** the connecting regions are formed as a form-fitting composite of material from the nonwoven layer (3) and solidified material of the backsheet film (1).

7. Arrangement according to one of claims 1 to 6, **characterized in that** the conductive imprint (2) is arranged between the backsheet film (1) and the nonwoven layer (3).

8. Arrangement according to one of claims 1 to 7, **characterized in that** the backsheet film (1) is not designed to be breathable, whereby the basis weight of the backsheet film (1) is less than 8 g/m², in particular less than 6 g/m².

9. Arrangement according to claim 8, **characterized in that**
the specific dart drop of the arrangement (4) is more than 6 g per gram of polymer per square meter
and/or
- the specific water column of the arrangement (4) is more than 270 mm per gram of polymer per square meter.

10. Arrangement according to claim 8 or 9, **characterized in that** the arrangement (4)
- has a force of at least 0.3 N/in per gram of polymer per m²,
- preferably a force of at least 0.375 N/in per gram of polymer per m²,
- in particular a force of more than 0.45 N/in per gram of polymer per m², in each case at 5% elongation.

11. Arrangement according to one of claims 1 to 7, **characterized in that** the backsheet film (1) is designed to be breathable, wherein the particular basis weight of the backsheet film (1) is less than 16 g/m², in particular less than 12 g/m².

12. Arrangement according to claim 11, **characterized in that**
- the specific dart drop of the backsheet film (1) is more than 26 g per gram of polymer per square meter and/or
- the specific water column of the backsheet film (1) is more than 460 mm per gram of polymer per square meter.

13. Arrangement according to claim 11 or 12, **characterized in that** the backsheet film (1) has a force of
- at least 0.5 N/in per gram of polymer per m²,
- preferably a force of more than 0.6 N/in per gram of polymer per m²,
- in particular a force of more than 0.7 N/in per gram of polymer per m²
in each case at 5% elongation.

14. Arrangement according to one of claims 1 to 13, **characterized in that** the conductive imprint (2) is arranged directly on the backsheet film (1).

## Revendications

1. Dispositif (4) pour la détection de liquide dans des articles hygiéniques jetables, **caractérisé en ce que** le dispositif (4) comprend une feuille de fond (1) avec une impression conductrice (2), le grammage de l'impression conductrice (2) étant inférieure à 3 g/m², la feuille de fond (1) n'est pas conçue pour être respirante et présentant un taux de transmission de la vapeur d'eau inférieure à 500 g/m² en 24 h selon la norme ASTM D6701-01, le grammage de la feuille de fond non respirante (1) étant inférieure à 10 g/m² ou la feuille de fond (1) est conçue pour être respirante et présente un taux de transmission de la vapeur d'eau supérieure à 500 g/m² en 24 h selon la norme ASTM D6701-01, le grammage de la feuille de fond (1) respirante étant inférieure à 20 g/m².

2. Dispositif selon la revendication 1, **caractérisé en ce que** l'impression conductrice (2) présente une résistance spécifique à la surface inférieure à 10 kΩ, de préférence inférieure à 6 kΩ, de préférence inférieure à 4 kΩ, en particulier inférieure à 1 kΩ.

3. Dispositif selon la revendication 1 ou 2, **caractérisé en ce que** le grammage de l'impression conductrice (2) étant inférieure à 2 g/m², de préférence inférieure à 1 g/m², en particulier inférieure à 0,5 g/m².

4. Dispositif selon l'une des revendications 1 à 3, **caractérisé en ce que** la feuille de fond (1) imprimée avec l'impression conductrice (2) ne présente aucune abrasion selon le test de frottement.

5. Dispositif selon l'une des revendications 1 à 4, **caractérisé en ce que** le dispositif (4) présente une couche non tissée (3), des zones de liaison étant disposées entre la couche non tissée (3) et la feuille de fond (1).

6. Dispositif selon la revendication 5, **caractérisé en ce que** les zones de liaison sont constituées d'un assemblage par complémentarité de forme à partir du matériau de la couche de non-tissé (3) et le matériau solidifié de la feuille de fond (1).

7. Dispositif selon l'une des revendications 1 à 6, **caractérisé en ce que** l'impression conductrice (2) est disposée entre la feuille de fond (1) et la couche non tissée (3).

8. Dispositif selon l'une des revendications 1 à 7, **caractérisé en ce que** la feuille de fond (1) n'est pas conçue pour être respirante, le grammage de la feuille de fond (1) étant inférieure à 8 g/m², en particulier inférieure à 6 g/m².

9. Dispositif selon la revendication 8, **caractérisé en ce que**
- la chute spécifique des fléchettes du dispositif (4) est supérieure à 6 g par gramme de polymère par mètre carré
et/ou
- la colonne d'eau spécifique du dispositif (4) est supérieure à 270 mm par gramme de polymère par mètre carré.

10. Dispositif selon la revendication 8 ou 9, **caractérisé en ce que** le dispositif (4) présente
- une force d'au moins 0,3 N/in par gramme de polymère par m²,
- de préférence une force d'au moins 0,375 N/in par gramme de polymère par m²,
- en particulier une force supérieure à 0,45 N/in par gramme de polymère par m²,
dans chaque cas avec un allongement de 5 %.

11. Dispositif selon l'une des revendications 1 à 7, **caractérisé en ce que** la feuille de fond (1) est conçue pour être respirante, le grammage de la feuille de fond (1) étant inférieure à 16 g/m², en particulier inférieure à 12 g/m².

12. Dispositif selon la revendication 11, **caractérisé en ce que**
- la chute de fléchette spécifique de la feuille de fond (1) est supérieure à 26 g par gramme de polymère par mètre carré et/ou
- la colonne d'eau spécifique de la feuille de fond (1) est supérieure à 460 mm par gramme de polymère par mètre carré.

13. Dispositif selon la revendication 11 ou 12, **caractérisé en ce que** la feuille de fond (1) présente une force de
- au moins 0,5 N/in par gramme de polymère par m²
- de préférence une force supérieure à 0,6 N/in par gramme de polymère par m²,
- en particulier une force supérieure à 0,7 N/in par gramme de polymère par m²
dans chaque cas avec un allongement de 5 %.

14. Dispositif selon l'une des revendications 1 à 13, **caractérisé en ce que**
l'impression conductrice (2) est disposée directement sur la feuille de fond (1).
